# EUROPEAN PATENT SPECIFICATION

(11) **EP 2 274 467 B1**
(45) Date of publication and mention of the grant of the patent: **27.02.2013**
(21) Application number: 09717393.4
(22) Date of filing: 06.03.2009
(51) Int. Cl.: D04H 1/4266, D04H 1/498, A61L 2/18, B60S 3/04, A47L 23/26

(54) **SANITARY MATS MADE OF NATURAL FIBRES AND METHOD OF OBTAINING SANITARY MATS**
SANITÄRMATTEN AUS NATURFASERN UND VERFAHREN ZUR HERSTELLUNG VON SANITÄRMATTEN
TAPIS SANITAIRES RÉALISÉS À PARTIR DE FIBRES NATURELLES ET PROCÉDÉ PERMETTANT D'OBTENIR DES TAPIS SANITAIRES

(30) Priority: 07.03.2008 PL 38463508
(43) Date of publication of application: 19.01.2011
(73) Proprietor: Instytut Wlokien Naturalnych I Roslin Zielarskich, 60-630 Poznan (PL)
(72) Inventor: MANKOWSKI, Jerzy, PL-61-308 Poznan (PL); KUBACKI, Andrzej, PL-82-200 Malbork (PL); KOLODZIEJ, Jacek, PL-60-652 Pozna (PL); KRASEMANN, Christian, 17279 Lychen (DE)
(74) Representative: Twardowska, Aleksandra
(86) International application number: PCT/PL2009/000020
(87) International publication number: WO 2009/110812

(56) References cited:
- EP-A1- 0 033 448
- EP-A1- 0 764 422
- WO-A1-00/20667
- WO-A1-91/08332
- WO-A1-97/05313
- DE-A1- 4 420 057
- DE-U1- 29 503 703
- FR-A1- 2 912 301
- US-A1- 2003 065 059
- US-A1- 2008 289 149

## Description

The subject of the invention is sanitary mats made of natural fibres and the method of obtaining sanitary mats. In order to minimise hazards caused by viruses and bacteria, it is very important to consequently implement the cleaning and disinfection programme, which is aimed at decreasing the infection hazard by taking the number of microorganisms present in the environment down below a certain secure level. The mat is used to disinfect vehicle wheels and pedestrian shoes.

It is not possible to give up high capacity animal animal production facilities, nor to lower animal production intensity or to lower the concentration degree per unit of area due to purely economic reasons. Therefore, it is necessary to provide the animals in production facilities with optimum living conditions. Research results indicate that despite high-scale vaccination within the frameworks of various pharmacological protection programmes, diseases caused by viruses and bacteria constitute the main reason for both animal losses and related economic ones. Therefore, all activities aimed at maintaining proper zoo-hygienic conditions in animal production facilities are very important.

Economic conditions at present, a need to minimise losses, and thus to lower costs in order to improve animal production efficiency, force us to achieve increasingly better results in animal production. It is only possible to achieve good results in animal production insofar as healthy animals may live in a healthy environment that is free of any hazards. Over the recent decades, many farms have emerged that require professional management, where a small mistake or negligence may lead to fatal financial results for the owner. Furthermore, a high degree of animal concentration at a unit of area in modem farms becomes an additional stress factor that affects animal immunity systems. Potentially, very high production capacity is achieved at the expense of lower overall immunity of animal organism to unfavourable external factors, including various infection types especially for young animals [Kazimierz G6rski, "Dezynfekcja, dezynsekcja i deratyzacja w chowie drobiu", Polskie drobiarstwo, no. 9/99]. ["Disinfection, disinsectisation and deratting in poultry breeding", Polish Poultry Breeding, no. 9/99].

Using appropriate preventive methods is one of the most important ways to fight diseases (including avian influenza and fowlpox or foot-and-mouth disease) placed by the World Organisation for Animal Health (OIE) at the A-list, which registers especially hazardous diseases that bring about the risk of rapid, dynamic spreading and serious social and economic consequences. It is one of the most critical issues that has been significantly emphasised since epidemic outbursts could be instrumental in causing immense, even disastrous losses.

Disinfecting mats are indispensable in slaughter houses, meat processing plants, food processing plants, veterinary clinics, infirmaries, animal quarantine stations, border crossings, farms, henneries, mushroom growing farms, foodstuff warehouses, zoological gardens, etc. In compliance with the Ordinance announced by the Minister of Agriculture and Rural Development, slaughter houses, meat processing plants, food processing plants and animal quarantine stations should be equipped with disinfecting mats.

In case of virus diseases that bring about the risk of rapid, dynamic spreading, in compliance with instructions announced by the Chief Veterinary Doctor, farmsteads should be secured (fenced) against any traffic of animals and people. The entire traffic should be limited to a single gate locked or barred with a barrier, at which appropriate, at least 3 meters long straw mats or mats made of some other material should be placed on the entire length of the entry (gate), and should be permanently kept moist by pouring a 2% caustic soda solution on them frequently and thoroughly. In frost conditions, salt should be added to disinfecting agents. To disinfect shoes, vessels (trays) should be placed at the entrance with a 2% caustic soda solution, whereas to disinfect hands and clothing, a tray with a 1% caustic soda solution and vessel with water, soap, disposable towel, etc. Entry into the areas (such as cow sheds, pigsties, sheep sheds, etc.), in which sick or suspected animals are present, only Veterinary Inspection employees and persons entrusted with taking care over these animals are allowed. At the entry to such areas (preferably inside them) disinfecting mats should be placed (plaited mats, bags filled with chaff, sawdust, etc.) together with vessels containing a 1% and 2% caustic soda solution, water, soap and a towel. All people who leave the premises should be thoroughly disinfected. [Chief Veterinary Doctor's Provisional Instruction of 7 March 2001 upon fighting the foot-and-mouth disease].

The first hours and days of an epidemic have a critical, decisive meaning. Most often, local entrepreneurs are unable to promptly react and meet the requirements that arise in the face of its rapid outburst. In this case, it is very important to prepare disinfecting agents and mats in advance, which is possible in case mats may be stored over long periods of time.

Quite frequently, checkpoints that control entry into infected zones, rather than a commercially available mat, place a plastic film covered with sawdust or straw, which is replaced as soon as it gets completely dirty or mudded. Equally often, however, disinfecting agent is wasted out by using straw or sawdust in these situations, which costs more than the mat itself.

Animal producers sometimes happen to disinfect their beddings (e.g. prior to bringing in chickens to their henneries), thus increasing their costs incommensurably to the effects they may reach. Furthermore, when disinfection is done by wet method using the sprinkling method, and the bedding is dried up, it poses a threat of lowering the quality of bedding as its moisture rises. At the same time, it is necessary here to assume that the bedding or the straw itself is of good quality, and that the producer knows the source of their origin and that he does not have to deal with low quality material that might be infected with mildew or fungi, which could be a threat to human and animal health.

In order to disinfect vehicle wheels and pedestrian shoes, Intermark Company offers its mats, which, upon having been soaked through with an appropriate disinfecting agent, may perform their function, that is to prevent transfer of hazardous microorganisms (bacteria, viruses) over long periods of time, i.e.: a) film-laminated Halomat on one side made of two layers of a sorptive nonwoven with HALOSORB mineral sorbent placed in between them. The unwoven fabric's lower layer that is film coated additionally secures the mat against the disinfecting substance leaking into the ground. Due to their properties, i.e. their high resistance to pressure and high chemical resistance, these mats are perfect for use in conditions of heavy vehicle and pedestrian traffic; b) polymer mats laminated on one side with a film that features high sorptive capability and high ease of giving away disinfecting agent that is contained in them; c) elastic polymer mats that are film-laminated on one side.

The Pesan Company produces disinfecting mats with polyurethane foam inside them. The way the mat is designed makes it impossible for the working liquid to leak out. Its top layer is a mesh that stops pollutants, and prevents the working liquid from evaporating too fast. When the mat is pressed, the working liquid gets through the mesh and disinfects shoes with no problem at all, and then it returns to the absorptive insert. Polyurethane foam thicknesses depend on the particular mat model. The mat absorbs the disinfecting liquid very well. It can be removed, washed, dried, or squeezed. Its PVC material is waterproof and resistant to high (+70°C) and low (-30°C) temperatures. It does not get damaged by weak acids or alkali. Just like the top mesh, it is easy to wash both by hand and using high pressure washing machines.

The M-tech Company produces disinfecting mats that are made of polyurethane foam, which is coated on its bottom and on its sizes with a layer that prevents the agent from leaking into the soil, and they constitute an independent disinfecting station. These mats should be soaked with a disinfecting agent, and this procedure should be repeated every day, so as to maintain their appropriate moisture and allow for effective disinfection of shoes and wheels.

CombiCleaner disinfecting mats are made on the outside of a highly durable polyester resistant to abrasion and low and high temperatures. Inside, a polypropylene foam insert can be found. One of the mat insert's characteristic features is its open-work construction, which allows, on one hand, for the disinfecting liquid to flow out under pressure, while on the other hand, prevents from pollutants getting inside the mat, and from evaporation of the liquid. The insert is situated inside an impermeable trough, from which it can easily be removed for cleaning purposes or to replace the disinfecting agent. The mat is not permanently fixed to the base, and thus its location may be easily changed as required. Thus, it constitutes an independent disinfecting station. Several mats can be used to form a disinfecting cycle, e.g. to disinfect vehicles.

One of CombiCleaner mats is a textile mat with nylon fibre type 6.6, which is dyed in its mass with antistatic additives, with thermally secured hair twist type *opi-twist.* Its fleece is resistant to crushing, and it enables the absorption of solid and liquid pollutants. The mat bottom is made of nitryl rubber (without the material memory), with some special outgrowths that closely adhere to the surface. Mats are finished with strengthened rubber edges, which prevent from the mat rolling up. The mat surface is sprayed with disinfecting agent. This procedure should be repeated every day, so that disinfecting mats may maintain their appropriate moisture. Mat maintenance [cleaning] can be conducted in drum washers or using the extraction method or by means of a washing vacuum-cleaner.

Patent Application no. KR20070078681 presents an antibacterial mat that contains bamboo sprout warps to provide favourable hygienic conditions by adding bamboo sprout warps (10%) to the filling material mass, and securing against bamboo sprouts getting broken by maintaining the appropriate moisture of bamboo sprout warps. The antibacterial mat (13) contains bamboo sprout warps, and contains an air-permeable covering woven fabric (15) and a filling material inside the covering woven fabric. The filling material contains washed and dried bamboo sprout warps as the base material, and bamboo sprout fibre warps obtained from the base material with antibacterial properties, which constitute more than 10% of the mat total weight. The moisture value of bamboo sprout warps that have been dried amounts to 5-15%.
Patent Application no. P-343828 (published on 10.09.2001) describes the mat that is made of vegetable fibres, preferably flax and hemp fibres, which is used in insulation applications, as well as the way this mat is produced. First, the fibres are pre-treated and shortened, and then they are divided in order to produce a fibre mass that contains single fibres whose lengths are contained within a desired interval. Then, the mat is produced of fibres in the dry forming process, whereas the fibres inside the mat are oriented at random. The dry forming process preferably takes place at the forming head that is situated over the vacuum box, whereas the fibres settle upon the forming wire in between these two elements. The plate becomes hardened, whereas bonds arise between individual fibres. Bonds between fibres may arise either using bonding agents or without using them. The mats formed in this way will be open and permeable, but at the same time stable and coherent enough to enable their handling.
Patent no. PL159294 (published on 10.07.1989) presents ways to produce the fibrous mat and the fibrous mat itself, which contains cellulose and thermoplastic fibres, especially polyester fibres and/or natural fibres of vegetable origin, as well as a hardenable bonding agent based on plastic material, whereas this fibrous mat may be used to obtain objects of permanent shape by pressing in the next technological operation by using heat and pressure, and as a result of hardening the bonding agent.

Despite extensive research devoted to obtaining disinfecting mats and to creating barriers that could be used to secure the environment against contamination and infection, as described above, there is still an urgent need for an effective solution that would make it possible to find a solution that would meet the above requirements, that would link in itself all the desirable features at the same time, and that would provide a possibility to promptly create barriers to cut off the zones that are infected or that are threatened to be infected. The necessity to use some additional preventive measures results from the ease with which pathogens can move from areas that have been infected to areas that are free from disease.

The objective of this invention is to deliver some means that could be used to obtain disinfecting mats from natural fibres that would be wet over relatively long periods of time, providing for much lower consumption of disinfecting agents as compared to the mats that are made of plastic materials and sawdust and bedding types that have been used to-date.

This invention meets the objective defined in the way as above, and resolves problems described in line with the to-date stand of technology, connected with obtaining products that may offer lower consumption levels of disinfecting agents, high durability levels, economic effectiveness and at the same time biodegradability, as well as aptness to create long disinfecting mat strips, so that the length of mats or basins can be higher than the perimeter of the biggest wheel of a vehicle that may pass over it, at the same time making it possible to promptly create such barriers to cut off the zones that are infected or that are threatened to be infected.

The subject matter of invention is a disinfecting mat, characterised in that it is obtained by processing fibrous plants, preferably hemp straw and green flax straw, using the decortication system, obtained by the process of claim 1. Preferably, the mat possesses high absorption characteristics of disinfecting agent solutions, at least 300% - 500%. The disinfecting agent solution is absorbed not only into areas in between the fibres, but it also permeates into the cell level of fibres, resulting in the so called hygroscopic moisture.
The disinfecting agent is applied to the mat as a water solution by pouring or spraying.
Preferably, the mat keeps its moisture for at least 2 to 4 days.
Preferably, the mat is biodegradable.
Preferably, it is manufactured from flax straw of some lower quality using the decortication system.
Preferably, it has high mechanic strength.
Preferably, it makes possible to promptly create a barrier to cut off the zones that are infected with a disease and/or zones that are threatened to be infected.

The next subject of invention is a method of obtaining a disinfecting mat, characterised in that the mat is made by processing fibrous plants using the decortication system, preferably using green hemp straw and/or flax straw, and then, upon cleaning pre-treatment, the fibres are fed into the roller carding machine, which straightens the fibres up by making them thinner, and then the fibres are formed into a carpet, and then, out of the carpet that has been formed by the carding machine in this way, the mat base is produced on jute fabric in several layers on the stacking machine, and then the base is thoroughly needled on the needling machine, thus creating a strong construction of the unwoven fabric.
The disinfecting agent solution is absorbed not only in areas in between fibres, but it also permeates into the cell level of fibres, resulting in the so called hygroscopic moisture.
The disinfecting agent is applied to the mat as a water solution by pouring or spraying.
Preferably, the mat is processed using the decortication system of flax straw of some lower quality.

The mat is produced by processing fibrous plants using the decortication system, preferably green hemp straw and/or flax straw, and then the fibres, upon cleaning pre-treatment, are fed towards the roller carding machine to be straightened up and made thinner, and then a carpet is formed, following which, out of the carpet that has been made by the carding machine in this way, the mat base is produced on the stacking machine on jute fabric in several layers, and the base is needled on the needling machine, thus creating a strong construction.
Disinfecting agent is applied to the mat as a water solution by pouring or spraying.
Below please find an exemplary implementation of this invention, which, hopefully, should make it easier to understand it.

### Example

Fibres to be used in the ecological production of disinfecting mats were obtained by processing green hemp straw, and, to a smaller degree, flax straw of some lower quality, using the decortication system. Fibres obtained in this way have high strength and elasticity. Mat strength is necessary for the mat to have high tensile strength, whereas its elasticity improves its absorptive characteristics. Upon cleaning pre-treatment, the fibres are fed into the roller carding machine. The carding machine divides fibre strands and straightens them up by making them thinner, and forms them into a carpet. Out of the carpet that has been made by the carding machine in this way, the mat base is produced by the stacking machine on jute fabric in several layers, and the carpet, together with the base, is thoroughly needled on the needling machine, to obtain a strong construction of the unwoven fabric.

The mat made in this way is approx. 6 mm thick and features an exceptionally high absorption degree of disinfecting solutions - approx. 400%. The mat that was 6 mm thick and 2 m² in area absorbed 8 litres of the disinfecting solution, which was a 9% caustic soda solution in this case. The disinfecting agent solution was absorbed not only to areas in between the fibres, but it also permeated into the cell level of fibres, resulting in the so called hygroscopic moisture, which was difficult to remove by squeezing, drying, absorbing, or washing out for instance by rainfall, and thus the mat was able to retain its disinfecting properties for 3 days in case of heavy traffic on the mat, and 4 days in case the traffic is less intensive. At the same time, from the mechanical point of view, the mat maintained its original properties and was not damaged for 3 months.

It is for reasons as above that the mat is able to retain its features of a disinfecting mat, by maintaining its moisture over relatively long periods of time. Natural raw materials used, as compared with plastic materials, result in the consumption level of disinfecting agents being much lower than in the case of plastic materials. The mat is used to disinfect vehicle wheels and pedestrian shoes.

The disinfecting agent in the form of a water solution is applied onto the mat by pouring or spraying. In case of this experiment, caustic soda solutions were used. Nevertheless, all available disinfecting agents are allowed.

Caustic soda in 9% solution is the most popular agent that has been used to-date.

1 kg of a dry mat is capable of absorbing 4 litres of the solution. Many different disinfecting agents are available in the market. Recommendations to use a specific agent should be submitted by a veterinary doctor who supervises the farm or another facility that requires a protective zone.

This agent should be used in line with its manufacturer's recommendations.

In order to improve mat quality, and above all to improve mat durability and to further decrease disinfecting agent consumption level, caoutchouc rubber coating will be used or a similar process on the bottom side, to reinforce and tighten the disinfecting agent outflow from the mat into the ground.

Disinfecting mats make it possible to promptly create barriers to cut off the zones that are infected or that are threatened to be infected.

## Claims

1. Process of obtaining the disinfecting mat, **characterised in that** the mat is made by processing fibrous plants in the decortication system, preferably using green hemp straw and/or flax straw, and then, upon cleaning pre-treatment, the fibres are fed into the roller carding machine, which straightens the fibres up by making them thinner, and then the fibres are formed into a carpet, and then, out of the carpet that has been formed by the carding machine in this way, the mat base is produced on jute fabric in several layers on the stacking machine, and then the base is thoroughly needled on the needling machine, thus creating a strong construction of the unwoven fabric and the disinfecting agent is applied to the mat as a water solution by pouring or spraying and wherein the disinfecting agent solution is absorbed not only in areas in between fibres, but it also permeates into the cell level of fibres.

2. Disinfecting mat obtained by the process of claim 1

3. Use of a disinfecting mat according to claim 2 to form promptly a barrier to cut off the zones that are infected with a disease and/or zones that are threatened to be infected.

## Patentansprüche

1. Verfahren zur Erzeugung einer Desinfektionsmatte, **gekennzeichnet dadurch, dass** die Matte durch Verarbeitung von Faserpflanzen im Dekortikationssystem hergestellt wird, vorzugsweise mit dem Einsatz von grünem Hanfstroh und/oder Leinenstroh, und anschließend während der reinigenden Vorbehandlung die Fasern in eine Walzenkardiermaschine eingespeist werden, die die Fasern gerade richtet und dünner macht, und nachdem die Fasern zu einem Vlies geformt worden sind, aus dem von der Kardiermaschine geformtem Vlies der Mattenboden hergestellt wird so, dass die Matte aus Jutegewebe in mehreren Schichten in der Stapelmaschine hergestellt, und anschließend vollständig mit der Nadelmaschine genadelt wird, so dass eine starke Konstruktion des Vliesstoffs erzeugt wird, wonach das Desinfektionsmittel in Form einer Wasserlösung durch begießen oder besprühen auf die Matte appliziert wird, wodurch die Desinfektionsmittellösung nicht nur in Bereichen zwischen den Fasern absorbiert wird, aber auch in die Zellenebene der Fasern eindringt.

2. Desinfektionsmatte, erzeugte im Verfahren nach Anspruch 1.

3. Anwendung einer Desinfektionsmatte nach Anspruch 2 zur schnellen Erzeugung einer Barriere, die mit einer Krankheit infizierte Zonen und/oder Zonen, die infiziert zu sein drohen, absperrt.

## Revendications

1. Procédé d'obtention du tapis de désinfection, **caractérisé en ce que** le tapis est fait par traitement des plantes fibreuses dans le système de décorticage, de préférence en utilisant de la paille de chanvre vert et/ou de la paille de lin, et puis, après traitement préalable de nettoyage, les fibres sont introduites dans la cardeuse à rouleaux, qui redresse les fibres en les rendant plus fines, et ensuite, les fibres sont formées en un tapis, et puis, à partir du tapis qui a été formé de cette façon par la cardeuse, la base du tapis est réalisée avec du tissu de jute en plusieurs couches sur la machine d'empilage, et ensuite, la base est aiguilletée complètement sur la machine d'aiguilletage, créant ainsi une construction solide du tissu non tissé, et l'agent désinfectant est appliqué sur le tapis en tant que solution d'eau, en le versant ou par pulvérisation, la solution de l'agent désinfectant étant absorbée non seulement dans les zones entre les libres, mais elle imprègne aussi le niveau cellulaire des fibres.

2. Tapis de désinfection obtenu par le procédé selon la revendication 1.

3. Utilisation d'un tapis de désinfection selon la revendication 2 pour former rapidement une barrière pour couper les zones qui sont contaminées par une maladie et/ou les zones qui sont menacées d'être contaminées.
